# EUROPEAN PATENT SPECIFICATION

(11) **EP 1 764 124 B1**
(45) Date of publication and mention of the grant of the patent: **19.01.2011**
(21) Application number: 06024468.8
(22) Date of filing: 10.12.2001
(51) Int. Cl.: A61M 25/00, A61B 5/0215, A61M 25/10, A61B 18/14, A61B 5/00

(54) **Intra-aortic balloon catheter having a fiberoptic sensor**
Intraaorttaler Balloonkatheter mit faseroptischem Sensor
Cathéter intra-aortique à ballonnet, pourvu d'un capteur à fibre optique

(30) Priority: 12.12.2000 US 734755; 09.08.2001 US 925143
(43) Date of publication of application: 21.03.2007
(62) Divisional of application: 04003691.5
(73) Proprietor: DATASCOPE INVESTMENT CORP., Montvale, N.J. 07645 (US)
(72) Inventor: Shock, Robert B., Sparta, NJ 07871 (US); Williams, Jonathan, Montville, NJ 07045 (US); Walters, Daniel A., Rockaway Township, NJ 07801-1918 (US)
(74) Representative: Schmidt, Karsten

(56) References cited:
- EP-A- 0 234 046
- EP-A- 0 307 162
- EP-A- 0 577 038
- EP-A- 0 609 914
- EP-A- 1 016 430
- JP-A- 2000 333 913
- US-A- 4 201 222
- US-A- 5 427 114
- US-A- 5 814 016
- US-A- 5 916 153

## Description

### BACKGROUND OF THE INVENTION

### Field of the Invention

The invention relates to a catheter having enhanced pressure sensing capabilities. More particularly, the invention relates to single and dual lumen intra-aortic balloon catheters having a fiberoptic sensor connected to the catheter.

### Description of the Prior Art

A key function of many catheters is that of continuously monitoring blood pressure. In many cases, this monitoring must be performed with accurate measurement of high frequency components. For example, reliable detection of the dicrotic notch of the aortic blood pressure waveform typically requires a pressure signal having a bandwidth of 15Hz or better. Detection of the dicrotic notch is generally used for the inflation/deflation timing of an intra-aortic balloon ("IAB") catheter.

Conventional invasive pressure monitoring is performed with low-cost fluid-filled transducers. A typical disposable monitoring kit, inclusive of all tubing, a continuous flush device, and a pre-calibrated transducer is very affordable. Unfortunately, these systems have several drawbacks. One major drawback is that bubbles or clots in the monitoring lines can reduce the frequency response of the system to a level below 15Hz, creating an "overdamped" condition. In other cases, the characteristics of the catheter and tubing can result in "ringing", which is associated with an underdamped condition. Furthermore, fluid-filled catheters can suffer from "catheter whip" (motion artifact), which is manifested as one or more high frequency deflections in the pressure signal. These problems can degrade the usefulness of the signal in applications such as intra-aortic balloon pumping (IABP). In particular, it is difficult, if not impossible, to automatically provide optimal timing of IABP using a pressure signal with a frequency response below 15Hz, or using signals with ringing or whip artifacts that mimic the physiologic dicrotic notch.

Another disadvantage of the traditional fluid-filled transducer is that it requires the use of an inner guidewire tube. Traditionally, IAB catheters have two lumens in the catheter: a gas shuttle lumen and an inner guidewire lumen. The gas shuttle lumen has to be large enough to allow the gas to shuttle back and forth without undue restriction to ensure speedy inflation and deflation of the IAB membrane. In order to make the catheter smaller it is desirable to remove the inner guidewire lumen or tube, thus requiring an alternate means for measuring arterial pressure.

Another means for monitoring blood pressure is to use a micromanometer, such as marketed by companies such as Millar, Endosonics, and Radi. See U.S. Patents Nos. 5,431,628 and 5,902,248. These devices can have excellent frequency responses, with system bandwidths greater that 200Hz. They are not subject to the negative effects of bubbles and catheter whip, and retain good performance even in the presence of small blood clots. Unfortunately, they are very expensive, prone to signal drift, and can suffer from electrical interference. A common source of electrical interference in the setting of IABP therapy is the use of electrosurgery. In this situation, it is desirable to maintain a reliable pressure signal with which to trigger the balloon, as the ECG signal which normally triggers IABP operation becomes completely unreliable. Conventional fluid-filled transducer systems are relatively immune from this type of interference. Attempts have been made to use micromanometers for IABP timing, see U.S. Patent Nos. 3,585,983 and 4,733,652. These attempts proved to be unreliable, as the device may be damaged during insertion and is also prone to signal drift. To address the drift issue, U.S. Patent no. 5,158,529 discloses a method for rezeroing the micromanometer by using the pressure from a partially filled balloon as it rests in the aorta. However, this method requires momentary interruption of IABP, which may be harmful to the critically ill patient.

While standard IAB catheters incorporating a fluid-filled transducer pressure measurement system or IAB catheters incorporating micromanometers may be suitable for the particular purpose employed, or for general use, they would not be as suitable for the purposes of the present invention as disclosed hereafter.
European Patent Application EP 0307 162 discloses an apparatus for positioning a sensor probe in vivo, including a probe advancement mechanism and a mating interconnection for positioning a distal end of an indwelling catheter. The distal tip of the probe is advanced through the catheter to a predetermined position with respect to the distal tip of the catheter at which the sensor measurement is made.
European Patent Application EP 0 234 046 discloses an intra-aortic balloon pump apparatus which comprises a catheter with a pressure transducer.
United States Published Patent 5,427,114 discloses an apparatus for determining pressure at one or a number of intermediate points along a catheter inserted into a closed cavity.

### SUMMARY OF THE INVENTION

Accordingly, there is a need for a reliable and affordable pressure monitoring approach that has high bandwidth pressure sensing, low signal drift, and freedom from electrosurgical interference. There is also a need to incorporate this technology into intra-aortic balloon catheters having small cross sectional profiles.

The invention is an IAB catheter system, with either a double or single lumen, having enhanced blood pressure sensing capability. The IAB catheter has a fiberoptic sensor built into the tip of the IAB or connected to another part of the catheter.
The fiberoptic sensor is for sensing blood pressure and includes a diaphragm over a chamber with a vacuum.

Fiberoptic sensors have the advantage of being immune to electrical interference, and have the potential to be lower in cost than electronic micromanometers.

To the accomplishment of the above and related objects the invention may be embodied in the form illustrated in the accompanying drawings. Attention is called to the fact, however, that the drawings are illustrative only. Variations are contemplated as being part of the invention, limited only by the scope of the claims.

### BRIEF DESCRIPTION OF THE DRAWINGS

In the drawings, like elements are depicted by like reference numerals. The drawings are briefly described as follows.

FIG 1 is a longitudinal cross sectional view of a distal end of a coaxial IAB catheter having a fiberoptic sensor in the tip.

FIG 1A is a perspective view of distal end of inner tube 58, shown independent of catheter 10, with pressure sensing line 24 connected to an outer surface of inner tube 58.

FIG 1B is a perspective view of a distal end of inner tube 58, shown independent of catheter 10, with pressure sensing line sandwiched between an outer surface of inner tube 58 and an outer layer.

FIG 1C is a perspective view of a distal end of inner tube 58, shown independent of catheter 10, with pressure sensing line 24 embedded in the wall of inner tube 58.

FIG 2 is a longitudinal cross sectional view of a distal end of a co-lumen IAB catheter having a fiberoptic sensor in the tip.

FIG 2A is a transverse cross sectional view of the co-lumen IAB catheter in FIG 2 taken along lines 2A-2A.

FIG 3A is a longitudinal cross sectional view of the tip having an embedded fiberoptic sensor in a first configuration.

FIG 3B is a longitudinal cross sectional view of the tip having an embedded fiberoptic sensor in a second configuration.

FIG 3C is a longitudinal cross sectional view of the tip having an embedded fiberoptic sensor in a third configuration.

FIG 3D is a longitudinal cross sectional view of the tip having an embedded fiberoptic sensor in a fourth configuration.

### DETAILED DESCRIPTION OF THE INVENTION

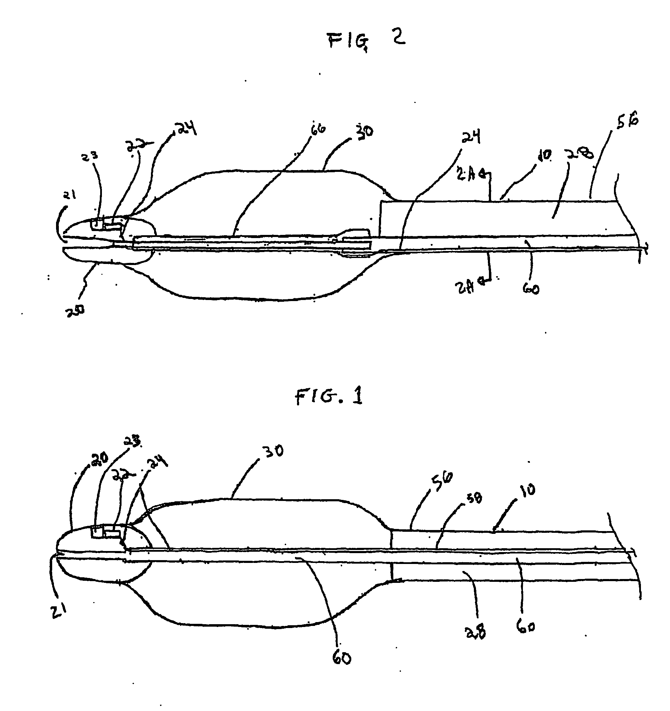

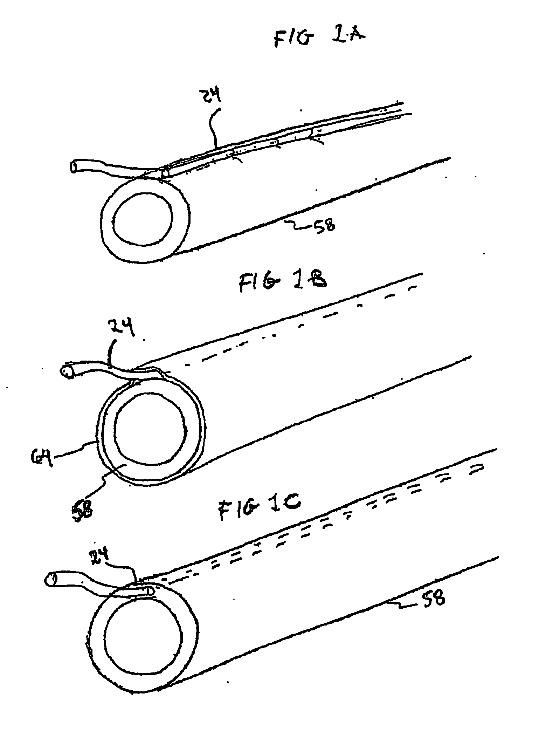

FIG 1 illustrates a longitudinal cross section of a distal portion of a dual lumen intra-aortic balloon ("IAB") catheter 10 comprising an outer tube 56, an inner tube 58, a tip 20, and a balloon membrane 30 connected on one end to the outer tube 56 and on the opposite end to the tip 20. Tip 20 defines a tip lumen 21. Inner tube 58 is disposed within the outer tube 56 and is connected to the tip 20 at its distal end. Inner tube 58 defines an inner lumen 60 and outer tube 56 defines an outer lumen 28. Outer lumen 28 is used for shuttling helium or another appropriate working gas or fluid for inflation and deflation of the balloon membrane 30. Outer tube 56 may be coil or braid reinforced and made from polyurethane or polyimide. Inner tube 58 may be made from polyimide or an alloy with shape memory and superelastic properties commonly referred to as Ni-Ti, NITINOL™, and other industry names. Inner tube 58 may be connected to an inner surface of the outer tube 56 at one or more points to enhance pushability, stability, pumping speed, and pressure fidelity.
Furthermore, inner tube 58 may be made from two or more tubes joined end-to-end, as disclosed in U.S. Patent No. 6,024,693, or may have an inner tube having a variable diameter, as disclosed in.U.S. Patent Application No. 09/764831, filed on January 17, 2001. Fiber optic sensor 22 is embedded in or attached to the tip 20 and preferably has a diameter less than or equal to (0.762 mm inches) 0.03. A forward looking end of the fiberoptic sensor 22 faces chamber 23. Pressure sensing line 24 may be affixed to inner tube 58 and connects fiber optic sensor 22 to an optoelectronic interface (not shown) via an industry-standard "SC" connector (not shown). Pressure sensing line 24 preferably is made of glass, but may be made of plastic or another material, and preferably has a diameter less than or equal to 0,152 mm (0.006 inches) Note that fiberoptic sensor 22 may be positioned in alternate locations along IAB catheter 10 as well as on a distal tip of an independent catheter that can be disposed within inner lumen 58.

FIGS 1A-1C illustrate perspective views of a distal end of inner tube 58 with pressure sensing line 24 connected to inner tube 58 in various configurations. In FIG 1A, pressure sensing line 24, is connected to an outer surface of inner tube 58. In FIG 1B, pressure sensing line 24 is disposed between inner tube 58 and a thin walled tube 64, which preferably is heat shrinkable. Heat shrinkable tubing with a wall thickness of less than or equal to (0.0254 mm inches) (0.001 mm) is available from Advanced Polymers, Inc., Salem, NH. In FIG 1C, pressure sensing line 24 is embedded in the wall of inner tube 58. Note that although pressure sensing line 24 is shown running along a longitudinal axis of inner tube 58 it may also be wound helically.

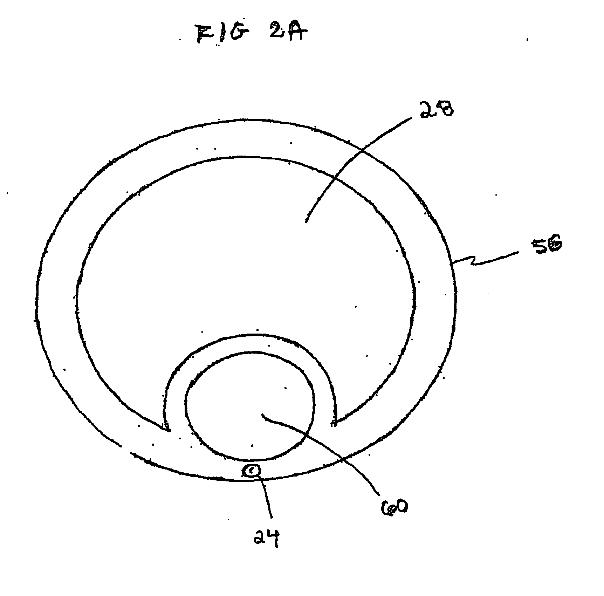

FIG 2 illustrates a distal portion of another embodiment of the IAB catheter 10, comprising a balloon membrane 30, a tip 20, a co-lumen tube 56, an inner lumen extension tube 66, and a fiberoptic sensor 22. Detailed structure of a co-lumen IAB is disclosed in U.S. Patent No. 6,024,693 and U.S. Patent Application Serial No .09/412, 718, filed on October 5, 1999.
Tip 20 is connected to a distal end of the balloon membrane 30 and to a distal end of the inner lumen extension tube 66. Tip 20 defines a tip lumen 21. A distal end of the co-lumen tube 56 is connected to a proximal end of the balloon membrane 30 and to a proximal end of the inner lumen extension tube 66. The co-lumen tube 56 may be coil or braid reinforced and made from polyurethane or polyimide. The preferred material for inner lumen extension tube 66 is an alloy with shape memory and superelastic properties commonly referred to as Ni-Ti, NITINOL™, and other industry names. Inner lumen extension tube 66 may also be made from polyimide. The fiberoptic sensor 22 is attached to tip 20 and pressure sensing line 24 which communicates signals generated by the fiberoptic sensor 22 to an optoelectronic interface (not shown). The pressure sensing line 24 may be connected to the inner lumen extension tube 66 and co-lumen tube 56 in any of the ways pressure sensing line 24 is connected to the inner tube 58, illustrated in FIGS 1A-1C. Furthermore, the pressure sensing line may be embedded in the outer tube 56 as illustrated in FIG 2A.

FIG 2A illustrates a transverse cross section of outer tube 56, taken along line 2A-2A illustrated in FIG 2, with pressure sensing line 24 embedded in the wall. Outer tube 56 defines two distinct lumens, inner lumen 60 and outer lumen 28. Outer lumen 28 is used for shuttling helium or another appropriate fluid or gas for inflation and deflation of balloon membrane 30. Note that pressure sensing line 24 may be embedded at a different location in outer tube 56, may be connected to a surface of outer tube 56, or may freely reside in outer lumen 28. Note also that the fiberoptic sensor 22 may be positioned in alternate locations along IAB catheter 10 as well as on a distal tip of an independent catheter that can be disposed within the inner lumen 60.

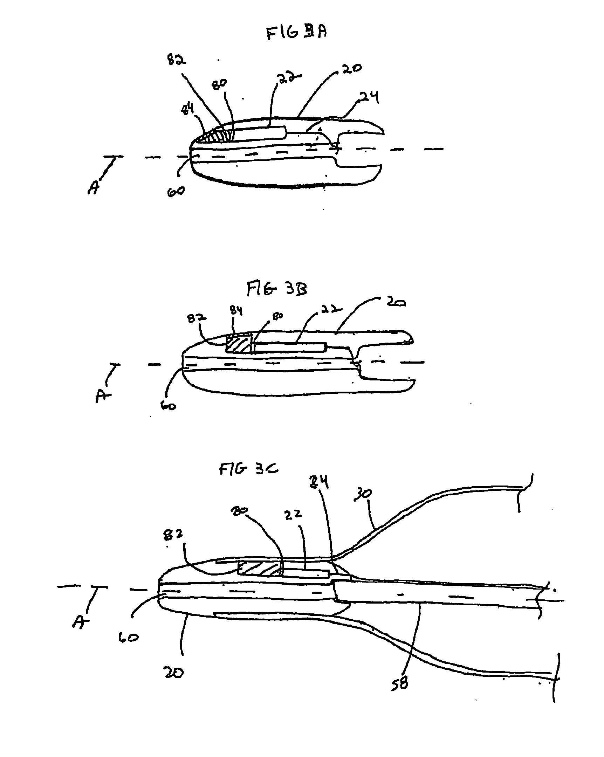

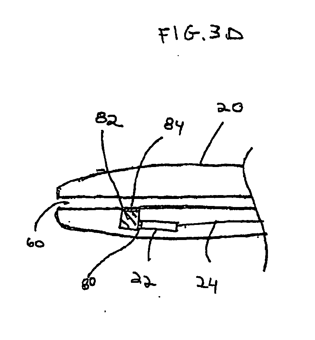

FIGS 3A-3D illustrate alternate configurations for tip 20 incorporating a forward looking fiber optic sensor 22. In FIG 3A and FIG 3B longitudinal cross sections of tip 20 are shown independent of the rest of catheter 10 for clarity. In FIG 3A fiber optic sensor is embedded in tip 20 parallel to the longitudinal axis of tip 20 labeled A. Fiber optic sensor 22 has an approximate outer diameter of 0.0635 mm (0.0025 inches) and has a pressure sensing surface or diaphragm 80 on a distal end which is exposed to protective pocket 82. Diaphragm 80 may be made from silicone approximately 6 microns thick and is protected from physical damage and blood contact by protective pocket 82, which may contain a gel, fluid, gas, elastomer, or any other flexible protective material. Diaphragm 80 may project into the pocket or may be flush with the protective pocket 82 wall. Membrane 84 prevents leakage of gel or protective pocket 82. Pressure sensing line 24 comprises a fiberoptic fiber having an outer diameter of approximately 0.152 mm (0.006 inches). In FIG 3B (and also FIGS 1 and 3) the gel or protective pocket 82 is shifted proximally. In FIG 3C a distal portion of balloon membrane 30 is used to seal protective pocket 82. In FIG 3D protective pocket 82 opens into inner lumen 60. Membrane 84 prevents leakage of gel out of protective pocket 82. In this location fiberoptic sensor 22 is exposed to arterial pressure via tip lumen 21 and is less likely to be damaged upon insertion and placement of IAB catheter 10. Note that although forward looking fiber optic pressure sensors are illustrated, use of side looking sensors is anticipated as well.

In addition to sensing blood pressure, the fiberoptic sensor 22 can be used to measure other important physiologic variables, such as, but not limited to temperature (as in the Luna Innovations sensor noted below), pCO2, pO2, pH, oxygen saturation, lactate, CPK, anesthetic agent concentration, various biological compounds, electrolytes, and nitric oxide. Blood pressure, as well as at least some of these variables, could be measured using either extrinsic or intrinsic fiberoptic sensors. Extrinsic sensors use the fiberoptic fiber to communicate information from a separate structure which is attached to the fiber. Intrinsic sensors sense information as a result of physical changes of the fiber itself. Intrinsic sensors have a particular advantage of being extremely small, rugged, and low in cost.

As indicated above the pressure sensing line 24 terminates in an optoelectronic interface which provides a light source, light detector, optical components (such as a lens, splitter, and interferometer), power supply, and signal processing means. This optoelectronic interface is known in the art and can be connected to or integrated with another electronic device, such as a monitor or intra-aortic balloon pump. Light originating from the interface travels down the fiberoptic fiber, i.e. the pressure sensing line 24, by the well-known principal of total internal reflection to the sensing element. The sensing element incorporates pressure responsive means which modifies the character of the light as a function of pressure. The modified light is in turn reflected back through the fiber to the interface. The optical components, light detector, and signal processing elements of the interface work together to produce an electrical output that characterizes the pressure at the sensor. This output is translated into a displayed pressure waveform or digital pressure display by the IABP or monitor.

Fiberoptic pressure monitoring sensors and optoelectronic interfaces suitable for this application are manufactured by several companies, including FISO Technologies Inc. (Quebec, Canada) and Luna Innovations (Blacksburg, VA). FISO utilizes a white light interferometric approach, as described in U.S. patents nos. 5,202,939 and 5,392,117.
Luna Innovations uses a laser light source in an approach similar to that described in U.S. patent no. 5,301,001.

There are a variety of possible configurations for the fiberoptic sensor itself. One type would employ a deformable diaphragm separated from the end of one or more fiberoptic fibers by a chamber. The chamber may be filled with air and vented to atmosphere to eliminate temperature-induced pressure changes within the chamber (these would be associated with signal drift). A disadvantage of this design in an IAB catheter is that an air-venting tube is required which occupies a portion of the cross-sectional area of the catheter. This tube reduces the potential to produce the catheter in a small size with acceptable performance.

A more desirable approach to a fiberoptic pressure sensor for a small catheter is to utilize a design without a vent tube (as illustrated in FIGS 1, 2, and 3A-3D). Such a sensor can be produced by positioning the sensor diaphragm over a chamber with a vacuum (as currently practiced by FISO). With this approach, a sensor can be constructed which has little temperature-induced drift, assuming that the materials which are used to build the diaphragm and chamber have small coefficients of thermal expansion (ceramics, glass, and silicon are good choices). This approach makes the device an absolute pressure sensor, which requires a separate barometric pressure sensor to convert absolute pressures to gauge pressures. In IABP applications, such a barometric pressure sensor could be built into the pump. U.S. Patent No. 5,158,529 teaches another possible means for assuring the accuracy of gauge pressure values as indicated by a sensor in the IAB catheter; this approach compares the blood pressure values indicated by the catheter sensor with pressure values in the gas within a partially-filled catheter and pump, and adjusts the sensor-indicated pressure to agree with the gas pressure.

Another approach to constructing a non-vented pressure sensor is to trap a gas in the chamber beneath the diaphragm and to monitor its temperature (as currently practiced by Luna Innovations). In this case, knowledge of the gas temperature within the chamber allows for correction of temperature-induced pressure changes. Ideally, if a gas is to occupy the chamber, it is dry and inert. It is possible that appropriate selection of the bias gas would improve linearization of the sensor or change its dynamic range.

## Claims

1. A balloon catheter system comprising a fiberoptic sensor catheter and a balloon catheter (10), said balloon catheter comprising a balloon membrane (30), a tip (20) having a tip lumen (21), an outer tube (56), and an inner tube (58) disposed within an outer surface of said outer tube, said inner tube extending beyond a distal end of the outer tube (56), a distal end of the balloon membrane (30) being connected to the tip (20) and to a distal end of the inner tube (58), said fiberoptic sensor catheter comprising a tube having a fiberoptic sensor (22) connected to a distal end, said fiberoptic sensor (22) being connected to a distal end of a fiberoptic fiber (24) which is connected to the tube, said fiberoptic sensor catheter fitting within the inner tube (58) and in the tip lumen (21), wherein said fiberoptic sensor (22) is for sensing blood pressure and includes a diaphragm over a chamber with a vacuum.

2. The balloon catheter system as claimed in claim 1 wherein the inner tube (58) is connected to the outer tube (56).

## Patentansprüche

1. Ballonkathetersystem, umfassend einen faseroptischen Sensorkatheter und einen Ballonkatheter (10), wobei der Ballonkatheter eine Ballonmembran (30), eine Spitze (20) mit einem Spitzenlumen (21), eine äußere Röhre (56) und eine innere Röhre (58), die in einer äußeren Oberfläche der äußeren Röhre angeordnet ist, umfasst, wobei sich die innere Röhre über ein Distalende der äußeren Röhre (56) hinaus erstreckt, wobei ein Distalende der Ballonmembran (30) mit der Spitze (20) und mit einem Distalende der inneren Röhre (58) verbunden ist, wobei der faseroptische Sensorkatheter eine Röhre umfasst, die einen faseroptischen Sensor (22) aufweist, der mit einem Distalende verbunden ist, wobei der faseroptische Sensor (22) mit einem Distalende einer Lichtleitfaser (24) verbunden ist, die mit der Röhre verbunden ist, wobei der faseroptische Sensorkatheter in die innere Röhre (58) und in das Spitzenlumen (21) passt, wobei der faseroptische Sensor (22) zum Messen des Blutdrucks gedacht ist und eine Membrane über einer Kammer mit einem Vakuum umfasst.

2. Ballonkathetersystem nach Anspruch 1, wobei die innere Röhre (58) mit der äußeren Röhre (56) verbunden ist.

## Revendications

1. Système de cathéter à ballon comprenant un cathéter avec capteur à fibre optique et un cathéter à ballon (10), ledit cathéter à ballon comprenant une membrane de ballon (30), une pointe (20) ayant une ouverture de pointe (21), un tube externe (56) et un tube interne (58) disposé sous une surface externe dudit tube externe, ledit tube interne s'étendant au-delà d'une extrémité distale du tube externe (56), une extrémité distale de la membrane du ballon (30) étant connectée à la pointe (20) et à une extrémité distale du tube interne (58), ledit cathéter avec capteur à fibre optique comprenant un tube ayant un capteur à fibre optique (22) connecté à une extrémité distale, ledit capteur à fibre optique (22) étant connecté à une extrémité distale d'une fibre optique (24) laquelle est connectée au tube, ledit cathéter avec capteur à fibre optique s'accommodant dans le tube interne (58) et dans l'ouverture de pointe (21), où ledit capteur à fibre optique (22) sert à détecter la pression artérielle et comprend un diaphragme sur une chambre sous vide.

2. Système de cathéter à ballon comme revendiqué dans la revendication 1 dans lequel le tube interne (58) est connecté au tube externe (56).
